Europäisches Patentamt

European Patent Office

Office européen des brevets

(11) Publication number: **0 208 783**
**A1**

# EUROPEAN PATENT APPLICATION

published in accordance with Art. 158(3) EPC

(21) Application number: 85900203.2

(22) Date of filing: 25.12.84

Data of the international appli-
cation taken as a basis:

(86) International application number:
**PCT/JP 84/00614**

(87) International publication number:
**WO 86/03740 (03.07.86 86/14)**

(51) Int. Cl.⁴: **C 07 C 67/00**, C 07 C 67/11,
C 07 C 69/007

(43) Date of publication of application: 21.01.87
**Bulletin 87/4**

(71) Applicant: **MITSUI TOATSU CHEMICALS,
INCORPORATED, 2-5 Kasumigaseki 3-chome,
Chiyoda-Ku Tokyo 100 (JP)**

(72) Inventor: **UENO, Kaoru, 5, Takiharu-cho, Minami-ku,
Nagoya-chi AICHI 457 (JP)**
Inventor: **HIRAYAMA, Yoshinobu, 99, Wakabadai,
Kani-shi Gifu 509-02 (JP)**
Inventor: **WATANABE, Yoshimoto, 3078-78,
Matsushin-cho 3-chome, Kasugai-shi AICHI 486 (JP)**
Inventor: **KAWAKAMI, Haruhiko, 4-19, Morigo-cho,
Atsuta-ku Nagoya-shi AICHI 456 (JP)**

(84) Designated Contracting States: **DE FR**

(74) Representative: **Schüler, Horst, Dr., Kaiserstrasse 41,
D-6000 Frankfurt/Main 1 (DE)**

(54) **PROCESS FOR PREPARAING ALLYL ESTERS.**

(57) A process for preparing allyl esters, which comprises
reacting a mixture composed mainly of allyl chloride and
chloropropenes, formed by the decomposition of 1,2-di-
chloropropane, with an alkali metal carbonate or with a
combination of a carboxylic acid and/or its anhydride and
an alkali metal carbonate.

DESCRIPTION

PRODUCTION PROCESS OF ALLYL ESTERS

Technical Field:

This invention relates to effective utilization of 1,2-dichloropropane. More specifically, it relates to a process for producing an allyl ester by reacting a decomposition mixture, which has been obtained by subjecting 1,2-dichloropropane to decomposition and principally comprises allyl chloride and chloropropenes, as is with a carboxylic acid.

Background Art:

1,2-Dichloropropane occurs as a by-product upon production of allyl chloride by the high-temperature chlorination of propylene or upon preparation of propylene oxide thorugh the chlorohydrination of propylene. In order to improve raw material consumption in the production of these allyl chloride and propylene oxide, it is essential to minimize the by-production of this 1,2-dichloropropane. Nevertheless, 1,2-dichloropropane is still by-produced in rather large amount. Thus, effective utilization of the thus-by-produced 1,2-dichloropropane is an important problem to be solved for improving raw material consumption in their production.

1,2-Dichloropropane is employed primarily as a solvent for 1,3-dichloropropane, a nematocide, and also as an economical solvent for paving asphalt, oil, grease and fat. There is, however, a problem that its use as a solvent is limited for safety consideration, since 1,2-dichloropropane contains the halogen.

Investigations have hitherto been conducted for the preparation of allyl chloride through the removal of hydrogen chloride from 1,2-dichloropropane. However, the removal of hydrogen chloride from 1,2-dichloropropane usually forms allyl chloride, 1-chloropropene (cis, trans) and 2-chloropropene, the separation and purification of which are difficult. For example, methods for isolating allyl chloride, which has high utility as an industrial raw material and occurs in a relatively large amount among such by-products (conversion: 50 - 90%; selectivity: 40 - 70%), from their mixture on industrial scales require more costs than the production cost of allyl chloride through the direct chlorination of propylene and are thus impractical.

It is, therefore, attempted to use the mixture as an industrial raw material without isolating them from one another. As one example, the chlorohydrinating reaction of the mixture may be mentioned. In this reaction, however, not only allyl chloride but also chloropropenes are subjected to the reaction to obtain a reaction product in the form of a mixture. Similar to the decomposition product of 1,2-dichloropropane, isolation and purification of the mixture are required.

Under the current circumstances, 1,2-dichloropropane are not effectively utilized as envisaged from the foregoing.

Disclosure of the Invention:

An object of this invention is to provide a process for making effective use on 1,2-dichloropropane which is by-produced in the production of allyl chloride by the high-temperature chlorination of propylene or in the preparation of propylene oxide through the chloro-hydrination of propylene.

Another object of this invention is to provide a process which is industrially advantageous for the production of allyl esters, useful compounds, making use of by-produced 1,2-dichloropropane.

These objects of this invention can be attained by the following process. Namely, an allyl ester may be obtained by subjecting 1,2-dichloropropane to thermal decomposition, reacting a thermally-decomposed mixture, which contains allyl chloride and chloropropenes formed by the thermal decomposition, with a carboxylic acid compound selected from alkali metal salts of a carboxylic acids or the combinations of carboxylic acids and/or carboxylic anhydrides and alkali metal carbonates so as to convert the allyl chloride primarily and selectively into the allyl ester, and then separating the allyl ester from the chloropropenes.

According to the process of this invention, it is possible to convert allyl chloride solely and selectively into an allyl ester and also to isolate chloropentenes by decomposing 1,2-dichloropropane and reacting the resultant decomposition mixture, which principally comprises allyl chloride and the chloropropenes, as is with a carboxylic acid compound.

In this process, it is advantageous from the industrial viewpoint to recycle unreacted allyl chloride and chloropropenes for their use after the allyl ester has been formed by the reaction between the decomposition mixture and the carboxylic acid compound. In this manner, the allyl chloride contained in the decomposition mixture of 1,2-dichloropropane can be converted almost completely into the allyl ester and at the same time, the chloropropenes can be isolated and obtained with high purity.

The selective conversion of allyl chloride into the allyl ester can be effected almost completely by recycling and reusing the unreacted mixture which contains several tens percent of allyl chloride. Thus, the recovery rate of allyl chloride can be improved significantly. Moreover, the thus-isolated chloropropenes are obtained with purity high enough to permit their industrial applications. Accordingly, the process of this invention permits industrially-advantageous use of 1,2-dichloropropane which has not conventionally been utilized effectively. Upon incorporation of water, preferably in an amount of 0.3 - 3.0 wt.%, in the decomposition mixture of 1,2-dichloropropane, stainless steel exhibits substantially perfect corrosion resistance to the reaction system. Thus, incorporation of such water allows to use an industrial-economical reaction apparatus.

Brief Description of the Drawing:

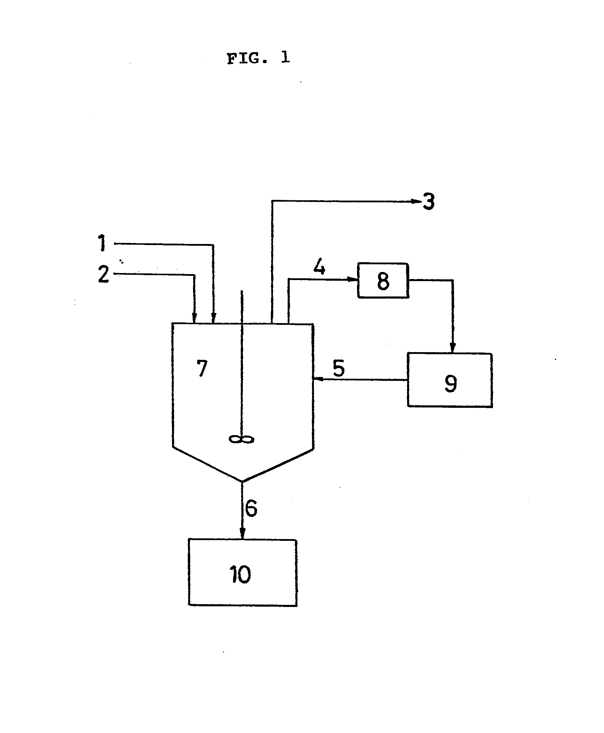

Figure 1 is a flow sheet for illustrating one embodiment of this invention.

## Best Mode for Carrying Out the Invention

The decomposition mixture of 1,2-dichloropropane useful in the practice of this invention can be obtained by either subjecting 1,2-dichloropropane to thermal decomposition either in the presence of a catalyst or in the absence thereof or reacting it with an alkali. Any mixtures obtained in accordance with processes other than those described above may also be used in the present invention, so long as such mixtures have the compositions similar to those of the decomposition mixtures obtained in the above-described manner.

Among carboxylic acid compounds useful in the practice of this invention, any alkali metal salt of a usual carboxylic acid may be employed as the alkali metal salt of the carboxylic acid. By way of example, may be mentioned alkali metal salts of phthalic acid, isophthalic acid, terephthalic acid, benzoic acid, naphthalenedicarboxylic acid, maleic acid, nitrophthalic acid, chlorobenzoic acid, succinic acid, cinnamic acid, glutaric acid, itaconic acid and the like.

Furthermore, as alkali metal salts of the above-described carboxylic acids their sodium and potassium salts are preferred.

In addition, it is also feasible to employ, instead of such an alkali metal salt of carboxylic acid, a carboxylic acid and/or carboxylic anhydride and an alkali metal carbonate, preferably, sodium or potassium carbonate.

The alkali metal salt of carboxylic acid or the carboxylic acid and/or carboxylic anhydride and the alkali metal carbonate, as well as the decomposition mixture of 1,2-dichloropropane are charged either at once or continuously and in the presence or absence of the catalyst so that the allyl chloride, which is contained in the decomposition mixture of 1,2-dichloro propane, is reacted.

In this invention, the amount of water which may be added to avoid the corrosion of a reaction apparatus may preferably range from 0.3 wt.% to 3.0 wt.% based on the decomposition mixture of 1,2-dichloropropane. Any amounts less than 0.3 wt.% cannot bring about enough corrosion resistance, whereas the yield is lowered due to decomposition of allyl chloride and the like if the water content exceeds 3.0 wt.%. It is, therefore, not preferred to add water in any amounts outside the above range.

The addition of water may be effected either at once in the initial stage of the reaction or continuously in the course of the reaction. From the viewpoint of effectiveness in avoiding corrosion, it is preferred to add it at once in the initial stage of the reaction.

Reaction conditions may vary depending on the type of the carboxylic acid compound. Generally speaking, it is however desirable to employ a reaction pressure of $0 - 50$ kg/cm$^2$G or particularly $0 - 10$ kg/cm$^2$G and a reaction temperature of $50 - 300°C$ or typically $120 - 200°C$. Incidentally, a solvent may be used unless it does not impair the reaction.

In addition, the reaction may be conducted while removing chloropropenes from the reaction system during the reaction. It is desirable to proceed with the reaction while charging the decomposition product of 1,2-dichloropropane, one of the raw materials, continuously and removing the chloropropenes continuously, because this manner permits use of a smaller reactor. Needless to say, the reaction may be carried out in a completely-sealed system, followed by the removal of unreacted chloropropenes after completion of the reaction.

Subsequent to the completion of the reaction, the resultant inorganic salts are filtered off and if necessary, the catalyst and unreacted allyl esters are separated and purified by extraction, distillation and/or the like.

In order to have allyl chloride, which is contained in the decomposition product of 1,2-dichloropropane, reacted with the carboxylic acid to such an extent that the allyl chloride is almost completely used up in such a fundamental reaction mode as described above, the reaction must be carried out, for example, (1) using the carboxylic acid compound and allyl chloride in the same equivalent amounts and choosing reaction conditions, or (2) using the carboxylic acid compound in an excess amount relative to the allyl chloride.

However, reliance on the approach (1) results in longer reaction time and at the same time, in a considerable reduction to the yield of the resulting allyl ester due to polymerization or the like of the allyl ester. On the other hand, the approach (2) leads to the carboxylic acid compound left over in an excessively large amount. Thus, the consumption of the carboxylic acid compound is increased and depending on the type of the carboxylic acid compound, extreme difficulty may be encountered upon separation of the unreacted carboxylic acid compound and the resultant allyl ester. The above approaches are accompanied by such problems as mentioned above.

These problems may however be solved by isolating allyl chloride and chloropropenes, which have been formed upon decomposition of 1,2-dichloropropane, as an allyl ester and as chloropropenes of high purity respectively, namely, (1) by reacting an unreacted mixture - which has in turn been obtained by reacting a decomposition mixture of 1,2-dichloropropane, said mixture principally comprising allyl chloride and chloropropenes, as is with a carboxylic acid compound and contains allyl chloride and chloropropenes primarily - with a carboxylic acid compound in an excess amount relative to the allyl chloride contained therein to obtain a reaction mixture containing an allyl ester, formed as a result of almost complete consumption of the allyl chloride, and unreacted carboxylic acid compound and at the same time to separate and obtain chloropropenes with high purity levels; and then (2) by reacting the reaction mixture with the decomposition product, which contains allyl chloride in at least the equivalent amount to the amount of the unreacted carboxylic acid compound contained in said reaction mixture, so as to convert the unreacted carboxylic acid contained in the reaction mixture into an allyl ester, thereby separating and recovering a second unreacted mixture which contains chloropropenes and allyl chloride primarily, and recycling and reusing the second unreacted mixture in the reaction with the carboxylic acid compound in the step (1).

One example of the practicing mode will be described in further detail with reference to Figure 1. The flow sheet of Figure 1 can be used to describe the production process of an allyl ester from 1,2-dichloro-propane. A carboxylic acid compound and catalyst are usually charged at once through a line 1 into a reactor 7 equipped with a stirrer. Thereafter, a decomposition mixture obtained by subjecting 1,2-dichloropropane to decomposition and principally comprising allyl chloride and chloropropenes is continuously fed through a line 2 to effect the reaction. Although reaction conditions vary depending on the type of the carboxylic acid compound used in the above reaction, the reaction pressure may be $0 - 50$ kg/cm$^2$G or preferably $0 - 10$ kg/cm$^2$G while the reaction temperature may be $50 - 300°C$ or preferably $120 - 200°C$.

Through the above reaction, the carboxylic acid compound is allowed to react primarily with allyl chloride in the decomposition mixture of 1,2-

0208783

dichloropropane, thereby selectively forming an allyl ester. On the other hand, an unreacted gas principally comprising chloropropenes and allyl chloride is removed through a line 4 either continuously during the reaction or at once after completion of the reaction. The unreacted gas is stored in a reservoir 9 by way of a condenser 8. On the other hand, the reaction product, i.e., the allyl ester is delivered through a line 6 to a tank 10. After filtering the resultant inorganic salt off and if necessary, recovering the catalyst and the like, the allyl ester is separated and purified by extraction, distillation and the like.

According to the above manner, the resultant allyl ester can be obtained as a final product. On the other hand, the thus-recovered first unreacted mixture still contains allyl chloride and chloropropenes which still remain unreacted. It is, therefore, extremely uneconomical to discard the first unreacted mixture without their recovery or to separate and recover them, respectively.

In the process of this invention, after reacting the decomposition mixture of 1,2-dichloropropane with the carboxylic acid in the above-described manner, a further reaction is conducted in the following manner using the first unreacted mixture which is stored in the reservoir 9 and contains allyl chloride and the chloropropenes.

Namely, the first unreacted mixture contains a substantial amount of allyl chloride which may be converted to the allyl ester.

Accordingly, the carboxylic acid compound, catalyst, etc. are charged at once through the line 1 in the same manner as described above. To the thus-charged contents, the first unreacted mixture stored in the reservoir 9 and containing the chloropropenes and allyl chloride is fed either continuously or at once. In this reaction, the carboxylic acid compound and unreacted mixture are fed to the reactor in such a way that the carboxylic acid compound is in at least the same equivalent amount as the allyl chloride contained in the unreacted mixture. An unreacted gas primarily comprising chloropropenes is discharged through the line 3 either continuously while proceeding with the reaction under the same conditions as mentioned above or at once after completion of the reaction, so that the chloropropenes are isolated. This discharge may be effected by a suction pump or the like, if necessary.

This reaction provides a reaction mixture containing the allyl ester, formed by the reaction of allyl chloride in the unreacted mixture with the carboxylic acid compound, and unreacted carboxylic acid compound as well as the chloropropenes substantially free of allyl chloride.

Furthermore, the decomposition mixture of 1,2-dichloropropane is charged through the line 2 either continuously or at once to the reaction mixture present in the reactor 7 so that they are reacted under the same reaction conditions as mentioned above. An unreacted mixture containing allyl chloride and the chloropropenes is drawn through the line 4, followed by its storage in the reservoir 9 by way of the condenser 8.

The thus-stored second unreacted mixture contains a substantial amount of unreacted allyl chloride along with the chloropropenes. It is thus recycled for its reuse to the reactor 7 in place of the first unreacted mixture.

The present invention will hereinafter be illustrated by Examples. Needless to say, this invention is by no means limited to the following Examples.

Referential Example:

(Thermal decomposition of 1,2-dichloropropane)

A heating wire was wound over a central 70 cm-long portion of a silica glass tube of 25 mm in internal diameter and 110 cm in length, thereby raising the internal temperature of the glass tube to 500°C. Through one end of the silica glass tube, 1,2-dichloropropene was charged at 350 g/hr. for its thermal decomposition in the tube. A decomposition product, which came out from the other end of the tube in a gaseous state, was condensed in a condenser cooled with ice water, thereby obtaining the decomposition mixture at 239 g/hr. The composition of the decomposition mixture was 47.2 wt.% of allyl chloride, 47.7 wt.% of 1-chloropropenes (as a mixture of its cis- and trans-isomers), 0.1 wt.% of benzene and 5.0 wt.% of unreacted 1,2-dichloropropane.

Example 1:

After placing 148 g (1 mole) of phthalic anhydride, 116 g (1.1 moles) of anhydrous sodium carbonate, 5 g of triethylamine, 1,4 g of water and 0.5 g of hydroquinone in an autoclave having an internal volume of 1 liter, the interior of the autoclave was purged with nitrogen and the contents were then heated to 140°C while stirring. Thereafter, 357 g (the content of allyl chloride: 168.4 g, 2.2 moles) of the thermal decomposition product of 1,2-dichloropropane, obtained in Referential Example 1, was gradually charged over 4 hours. The reaction was then continued for 2 hours. While maintaining the reaction temperature at 140°C, the resulting carbon dioxide gas and unreacted chloropropenes were gradually taken out of the autoclave to control the reaction pressure at 10 kg/cm$^2$G, and were discharged via a dry ice trap to the outside of the reaction system. After completion of the reaction, the internal pressure of the autoclave was gradually reduced to normal pressure and at the same time, the autoclave was cooled to room temperature. Then, the contents were taken out of the autoclave, the resultant inorganic matter was filtered off, the filtration residue was washed with ether, and the filtrate and ether washing were combined together. After washing the ether solution with water to remove water-soluble matter, the resultant ether solution was dried with anhydrous sodium sulfate and the ether was removed by its distillation under reduced pressure, thereby obtaining 227 g (0.92 moles) of diallyl phthalate.

Example 2:

The procedures of Example 1 were repeated except that in Example 1, 166 g (1 mole) of isophthalic acid was used instead of phthalic anhydride and the amount of triethylamine was increased to 10 g, thereby obtaining 173.7 g (0.705 mole) of diallyl isophthalate.

Example 3:

Charged in an autoclave having an internal volume of 1 liter were 324 g (2 moles) of sodium benzoate monohydrate, 8 g of triethylamine and 0.5 g of hydroquinone. After purging the autoclave with nitrogen, the contents were heated to 130°C while stirring. Then, 341 g (the content of allyl chloride: 161 g, 2.1 moles) of the decomposition product of 1,2-dichloropropane, obtained in Referential Example 1, was gradually charged over 4 hours. Thereafter, the reaction was continued for further 2 hours. While maintaining the reaction temperature at 130 - 140°C, unreacted chloropropenes were discharged via a dry ice trap in such a way that the reaction pressure was controlled at 6 kg/cm$^2$G. After completion of the reaction, the internal pressure of the autoclave was gradually reduced to normal pressure and at the same time, the autoclave was cooled to room temperature. Then, the contents were taken out of the autoclave, the resultant inorganic matter was filtered off, the filtration residue was washed with ether, and the filtrate and ether washing were combined together. After washing the ether solution with water to remove water-soluble matter, the resultant ether solution was dried with anhydrous sodium sulfate and the ether was removed by its distillation under reduced pressure, followed by an addition of 0.5 g of hydroquinone to the residue. The resulting solution was then distilled under reduced pressure to obtain 298 g (1.84 moles) of allyl benzoate.

Example 4:

After charging 148 g (1 mole) of phthalic anhydride, 116 g (1.1 moles) of anhydrous sodium carbonate, 5 g of triethylamine, 0.5 g of hydroquinone, 3.0 g of water and stainless steel, SUS 304 (3.1 x 2.5 x 0.17 cm, 9.1810 g) as a specimen in an autoclave having an internal volume of 1 liter, the interior of the autoclave was purged with nitrogen and the contents were then heated to 140°C while stirring.

Thereafter, 357 g (the content of allyl chloride: 168.4 g, 2.2 moles) of the thermal decomposition product of 1,2-dichloropropane, obtained in the Referential Example 1, was gradually charged over 4 hours. Then, the reaction was continued for further 2 hours.

While maintaining the reaction temperature at 140°C, the resulting carbon dioxide gas and unreacted chloropropenes were gradually removed from the autoclave so as to maintain the reaction pressure at 10 kg/cm$^2$G. They were discharged to the outside of the reaction system through a dry ice trap. After completion of the reaction, the pressure within the autoclave was reduced to normal pressure and at the same time, the autoclave was cooled to room temperature. Then, the yellowish white and viscous liquid contents were taken out of the autoclave. The resultant inorganic matter was filtered off from the contents, the filtration residue was washed with water, and the filtrate and ether washing were combined together, After washing the ether solution with water and removing water-soluble matter, the ether solution was dried with anhydrous sodium sulfate and the ether was removed by its distillation under reduced pressure to obtain 227 g (0.92 mole) of diallyl phthalate.

On the other hand, the weight loss of the stainless specimen was 0.0001 g. Its corrosion rate was thus 0.01 mm/year.

Comparative Example 1:

The procedures of Example 4 were repeated without the addition of water. After completion of the reaction, the contents were in the form of dark greenish and viscous liquid. 225 (0.91 mole) of diallyl phthalate was obtained.

On the other hand, corroded pores were observed in the surfaces of the stainless specimen. Its weight loss was 0.3130 g and its corrosion rate was 32.9 mm/year.

Example 5:

(1) Thermal decomposition of 1,2-dichloropropane:

The procedures of Referential Example 1 were followed to obtain a decomposition mixture.

(2) Production of an allyl ester:

After charging 148 g (1 mole) of phthalic anhydride, 116 g (1.1 moles) of anhydrous sodium carbonate, 5 g of triethylamine, 1.4 g of water and 0.5 g of hydroquinone in an autoclave having an internal volume of 1 liter, the interior of the autoclave was purged with nitrogen and the contents were then heated to 140°C while stirring. Thereafter, 357 g (the content of allyl chloride: 168.4 g, 2.2 moles) of the above decomposition mixture of 1,2-dichloropropane was gradually charged over 4 hours. The reaction was continued for further 2 hours. While maintaining the reaction temperature at 140°C, the resulting carbon dioxide gas and unreacted allyl chloride and chloropropenes were gradually removed from the autoclave to maintain the reaction pressure at 10 kg/cm$^2$G and were discharged to the outside of the reaction system through a dry ice trap. The composition of the unreacted mixture recovered in the dry ice trap at this stage was 19.8 wt.% of allyl chloride and 80.2 wt.% of 1-chloropropene (as a mixture of its cis- and trans-isomers).

After completion of the reaction, the internal pressure of the autoclave was gradually lowered to normal pressure and at the same time, the autoclave was cooled to room temperature. Then, the contents were taken out of the autoclave, the resultant inorganic matter was filtered off, the filtration residue was washed with ether, and the filtrate and ether washing were combined together. After washing the ether solution with water and removing water-soluble matter, the ether solution was dried with anhydrous sodium sulfate and the ether was removed by its distillation under reduced pressure to obtain 227 g (0.92 mole) of diallyl phthalate.

(3) Reaction between the unreacted mixture and carboxylic acid:

After charging 148 g (1 mole) of phthalic anhydride, 116 g (1.1 moles) of anhydrous sodium carbonate 5 g of triethylamine, 1.4 g of water and 0.5 g of hydroquinone in an autoclave having an internal volume of 1 liter, the interior of the autoclave was purged with nitrogen and the contents were then heated to 140°C while stirring. Thereafter, 193 g (the content of allyl chloride: 38.3 g, 0.5 mole) of the recovered unreacted mixture obtained in the above step (2) was gradually charged over 4 hours. The reaction was continued for further 2 hours. While maintaining the reaction temperature and at 140°C, the resulting carbon dioxide gas and unreacted chloropropenes were gradually removed from the autoclave to maintain the reaction pressure at 10 kg/cm$^2$G and were discharged to the outside of the reaction system through a dry ice trap. At this stage, 1-chloropropene containing 0.3 wt.% of unreacted allyl chloride was obtained in the dry ice trap.

(4) Reaction between the reaction mixture and thermal decomposition product:

After completion of the reaction, the internal pressure of the autoclave was gradually lowered to normal pressure. To the reaction mixture containing diallyl phthalate and unreacted phthalic anhydride, was gradually added over 4 hours 324 g (the content of allyl chloride: 153 g, 2.0 moles) of the thermal decomposition mixture of 1,2-dichloropropane, which was obtained in the above step (1). The reaction was continued for further 2 hours. While maintaining the reaction temperature at 140°C, the resulting carbon dioxide gas and unreacted allyl chloride and chloropropenes were gradually removed from the autoclave to maintain the reaction pressure at 10 $kg/cm^2G$ and were then discharged via a dry ice trap to the outside of the reaction system. The composition of the unreacted mixture recovered in the dry ice trap was 20.0 wt.% of allyl chloride and 80.0 wt.% of 1-chloropropene (as a mixture of its cis- and trans-isomers).

After completion of the reaction, the internal pressure of the autoclave was gradually lowered to normal pressure and at the same time, the autoclave was cooled to room temperature. The resultant reaction mixture was then purified in the same manner as in the above step (2), thereby obtaining 236 g (0.96 mole) of diallyl phthalate.

(5) Reuse of the unreacted mixture:

The unreacted mixture recovered in the dry ice trap was used for the same reaction as in the step (3).

Similar results were obtained.

## CLAIMS

1. A process for producing an allyl ester, which comprises reacting a decomposition mixture, which has been obtianed by subjecting 1,2-dichloropropane to decomposition and principally includes allyl chloride and chloropropenes, as is with a carboxylic acid compound selected from alkali metal salts of carboxylic acids or combinations of carboxylic acids and/or carboxylic anhydrides and alkali metal carbonates.

2. A process according to Claim 1, wherein the carboxylic acid compound is an alkali metal salt of phthalic acid, isophthalic acid, terephthalic acid, benzoic acid, naphthalenedicarboxylic acid, maleic acid, nitrophthalic acid, chlorobenzoic acid, succinic acid, cinnamic acid, gluraric acid or itaconic acid.

3. A process according to Claim 1, wherein the alkali metal salt of the carboxylic acid is a sodium or potassium salt of the carboxylic acid.

4. A process according to Claim 1, wherein the carboxylic acid compound is a combination of phthalic acid, isophthalic acid, terephthalic acid, benzoic acid, naphthalenedicarboxylic acid, maleic acid, nitrophthalic acid, chlorobenzoic acid, succinic acid, cinnamic acid, glutaric acid or itaconic acid or the anhydride thereof and an alkali metal carbonate.

5. A process according to Claim 1, wherein the alkali metal carbonate is sodium or potassium carbonate.

6. A process according to Claim 1, wherein water is added in an amount of 0.3 - 3.0 wt. % based on the decomposition mixture.

7. A process according to Claim 1, wherein the reaction is conducted at a gauge pressure of 0 - 50 $kg/cm^2$ and at a temperature of 50 - 300°C.

8. A process according to Claim 7, wherein the reaction is conducted at a gauge pressure of 0 - 10 $kg/cm^2$ and at a temperature of 120 - 200°C.

9. A process according to Claim 1, wherein said process comprises:

(a) reacting a first unreacted mixture, which has been obtained upon the reaction between the decomposition mixture and carboxylic acid compound and contains allyl chloride and chloropropenes primarily, with the carboxylic acid compound in at least the equivalent amount to the allyl chloride contained in the first unreacted mixture, followed by separation into a reaction mixture, which contains the resulting allyl esters and unreacted carboxylic compound, and the chloropropenes;

(b) then, reacting the reaction mixture with the decomposition mixture, which contains the allyl chloride in at least the equivalent amount to the unreacted carboxylic acid compound contained in the reaction mixture, followed by separation into resultant allyl esters and a second unreacted mixture containing allyl chloride and chloropropenes; and

(c) recycling the second unreacted mixture for a reaction with the carboxylic acid compound in the step (a).

0208783

1 / 1

FIG. 1

# INTERNATIONAL SEARCH REPORT

International Application No. PCT/JP84/00614

## I. CLASSIFICATION OF SUBJECT MATTER (if several classification symbols apply, indicate all) [3]

According to International Patent Classification (IPC) or to both National Classification and IPC

Int. Cl$^4$ C07C 67/00, 67/11, 69/007

## II. FIELDS SEARCHED

### Minimum Documentation Searched [4]

| Classification System | Classification Symbols |
|---|---|
| IPC | C07C 17/34, 17/38, 19/02, 21/04, 21/067, 67/00, 67/10, 67/11, 69/007, 69/025, 69/155, 69/76, 69/78, 69/80 |

Documentation Searched other than Minimum Documentation
to the Extent that such Documents are Included in the Fields Searched [5]

## III. DOCUMENTS CONSIDERED TO BE RELEVANT [14]

| Category* | Citation of Document, [16] with Indication, where appropriate, of the relevant passages [17] | Relevant to Claim No. [18] |
|---|---|---|
| A | JP, A, 49-62407 (Teijin Ltd.) 17 June 1974 (17. 06. 74) Claim (Family nashi) | 1 - 9 |
| A | JP, A, 50-105611 (Osaka Soda Co., Ltd.) 20 August 1975 (20. 08. 75) Claim (Family nashi) | 1 - 9 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| March 20, 1985   (20. 03. 85) | April 1, 1985   (01. 04. 85) |

| International Searching Authority [1] | Signature of Authorized Officer [20] |
|---|---|
| Japanese Patent Office | |

Form PCT/ISA/210 (second sheet) (October 1981)